# EUROPEAN PATENT APPLICATION

(11) **EP 3 971 303 A1**
(43) Date of publication of application: **23.03.2022**
(21) Application number: 20197293.2
(22) Date of filing: 21.09.2020
(51) Int. Cl.: C12Q 1/6848, B01L 3/00, G01N 21/25

(54) **A METALLIC SAMPLE HOLDER, A PROBE FOR PERFORMING DETECTION REACTIONS, AND APPARATUS FOR RECEIVING THE SAMPLE HOLDER**

(71) Applicant: ETH Zurich, 8092 Zürich (CH)
(72) Inventor: STARK, Wendelin Jan, 4900 Langenthal (CH); GREGORINI, Michele, 4656 Starrkirch-Will (CH); BECHTOLD, Philippe, 8049 Zürich (CH)
(74) Representative: Rey, Antje Gabriele

(57) **Abstract**

A metallic sample holder 1 comprising an array of wells 10, wherein each well of the array is adapted to capture a sample volume. A first sector 151 of the array comprises wells that are loaded with a first reagent of a first freeze-dried nucleic acid amplification test reagent. A second sector 152 of the array comprises wells that are loaded with a second reagent of a second freeze-dried nucleic acid amplification test reagent.

## Description

### Technical Field

The present invention relates to a metallic sample holder, an apparatus adapted to receive the metallic sample holder, a method for detection by means of the metallic sample holder, and a use of the apparatus.

### Background Art

Nucleic acid amplification tests (NAAT) are techniques to reproduce a particular nucleic acid sequence numerous times and thus detect them. There are many techniques of nucleic acid amplification, such as Strand displacement amplification (SDA), Transcription-mediated amplification (TMA), Loop-mediated isothermal amplification (LAMP) and Polymerase chain reaction (PCR) In particular, Polymerase chain reaction (PCR) is a technique used in molecular biology for repeatedly replicating focused segments of deoxyribonucleic acid molecules (DNS). In particular, such a reaction process relies on thermal cycling of the samples, involving exposure of the samples to cycles of repeated heating and cooling. Thermal cycling can be utilized to provide heating and cooling of multiple reaction vessels, which may contain biological and/or chemical substances in order to carry out specific reactions.

Such NAAT techniques are well known in the field. For example, one of these, namely the polymerase chain reaction techniques are disclosed in US 4'683'202.

There are many different types of apparatuses for performing detection of NAAT. One of the essential components of such an apparatus is the sample holder. Conventionally, thermal cycling devices include a thermal block that is designed to capture tubes or plates that carry the sample volumes.

One or more Peltier units are used to heat or cool the thermal block that has a thermal mass at least one hundred times larger than the thermal mass of sample volumes captured within the tubes or plates.

Moreover, the cooling or heating capacity of the Peltier elements depends mainly on the cooling respectively heating rate of the thermal block. The time that it takes for cooling or heating the large thermal block is therefore significant for the duration of the whole measurement.

In addition, it is desirable to control the temperature change of the sample volumes in a manner that accurately attains the target temperature, avoids undershooting or overshooting of the temperature, and quickly reaches the target temperature. Such control of temperature may inhibit side reactions, the formation of unwanted bubbles, the degradation of components at certain temperatures, etc., which may occur at non-optimal temperatures. The temperature of the sample volumes that are captured in the tubes or plates can nearly not be monitored for this type of sample holder, since it might deviate from the temperature of the thermal block.

### Disclosure of the Invention

The problem to be solved by the present invention is therefore to provide a sample holder, a probe for performing detection reactions, and an apparatus adapted for receiving the metallic sample holder that overcome the disadvantages of the prior art.

The problem is solved by the subjects of the independent claims. Accordingly, a **first aspect** of the invention concerns a metallic sample holder.

Advantageously, the metallic sample holder is adapted for capturing sample volumes for nucleic acid amplification test reactions.

The sample holder comprises an array of wells.

Each well of the array is adapted to capture a sample volume. A first sector of the array comprises wells that are loaded with a first freeze-dried nucleic acid amplification test kit and a second sector of the array comprises wells that are loaded with a second freeze-dried nucleic acid amplification test kit.

In particular, the test kit refers to a mix of reagents comprising at least one set of primers for the detection or identification or quantification of nucleic acids.

Advantageously, the metallic sample holder is configured to capture sample volumes for nucleic acid amplification (NAA) detection, in particular polymerase chain reaction (PCR) detection.

The wells are advantageously arranged in a regular pattern to enable facile preparation of the samples, in particular by means of automated preparation. In particular, the sample volumes are directly filled into the wells. Therefore, the preferably liquid sample volumes are directly in touch with the sample holder surface within the wells.

In a further advantageous embodiment, the wells are arranged in a very specific pattern, to provide a unique array, e.g. for applying specific identification mechanisms on the particular array. Such identification methods might be correlated with a specific arrangement of the wells.

Advantageously, the wells correspond to so called sample wells, wherein each well corresponds to one sample well. The terms "well" and "indentation" are used here synonymously.

The term indentations refers therefore in particular to cavities in the sample holder. Preferably, these indentations are formed by removing material from a plate representing the sample holder yet without wells. In particular, the term wells can further refer to recesses in the sample holder

In an advantageous embodiment of the invention, forms of sample volumes or reaction sites according to embodiments of the present invention may include reaction volumes located within wells or wells formed in a substrate, spots of solution distributed on the surface a substrate, or other types of reaction chambers or formats.

The first and/or second sector can be defined to separate the wells of the array in an equal or unequal number.

In a further advantageous embodiment of the invention, the array can comprise further sectors, in particular the array can comprise a third and fourth and fifth sector with a corresponding third, fourth or fifth test kit for detecting a corresponding third, fourth or fifth nucleic acid sequence.

But, in a further advantageous embodiment of the invention, the array can comprise exactly two sectors, wherein the wells of the array are equally distributed between the two sectors.

Advantageously, the freeze-drying of the equipment includes: in the pre-freezing phase, the temperature of the separator drops to -55 ° C, the holding time during pre-freezing is 1h, and then the equipment is evacuated to maintain freeze-drying for 1h; in the analytical drying stage The temperature was raised to -25 ° C for 1h, then the temperature of the separator was increased to 37 ° C and maintained for 2h, and finally the separator was lowered to 25 ° C and maintained for 1h.

In particular, there might be a set of sample holders, wherein for each set of the sample holders, the first and second sector of the array are specifically allocated. The allocation of the first and second sector of the array might be the same for each sample holder of the set or might vary from sample holder to sample holder of the set.

In a further advantageous embodiment of the invention, the first test kit comprises a first set of primers specific for a first test for identifying and/or quantifying a first nucleic acid, in particular a first virus. The second test kit comprises a second set of primers specific for a second test for identifying and/or quantifying a second nucleic acid, in particular a second virus.

In a further advantageous embodiment of the metallic sample holder, the first test kit comprises additionally a nucleic acid amplification enzyme, dNTPs, and a buffer.

In particular, the first test kit can further comprise a fluorescent dye or a nucleic acid detection probe, such as a TaqMan probe,

Advantageously, also the second test kit can additionally comprise a nucleic acid amplification enzyme, dNTPs, and a buffer.

In particular, the second test kit can further comprise a fluorescent dye or a nucleic acid detection probe, such as a TaqMan probe.

In an advantageous embodiment, the first test kit comprises a reverse transcriptase enzyme; and/or the second test kit comprises a reverse transcriptase enzyme.

In a further advantageous embodiment, the first nucleic acid is a first RNA or DNA isolated from a first virus, in particular from a corona virus; and/or
the second nucleic acid is a second RNA or DNA isolated from a second virus, in particular an influenza virus.

In a further advantageous embodiment, the first nucleic acid is a first RNA or DNA isolated from a first virus, in particular from a SARS-CoV-2 virus; and/or

the second nucleic acid is a second RNA or DNA isolated from a second virus, in particular an influenza virus.

In a further advantageous embodiment of the invention, the first test kit comprises an internal reference dye; and/or the second test kit comprises an internal reference dye.

In a further advantageous embodiment of the invention, the first test kit of the metallic sample holder comprises the first set of primers specific for detection of a corona virus.

In a further advantageous embodiment of the invention, the second test kit comprises a second set of primers specific for detection of an influenza virus.

The advantageous sample holder with freeze-dried nucleic acid amplification test (NAAT) reagents for the simultaneous detection of the coronavirus and the seasonally predominant influenza virus includes primers and probes.

Advantageously, the freeze-drying includes the following steps: placing the sample holder filled with the fluorescent NAAT reaction system at -80 ° C for 1 hour, and then freeze-drying the sample holder

In a further advantageous embodiment of the invention, each well is adapted to capture a maximal sample volume vₘₐₓ, with vₘₐₓ = 15 µl, in particular with vₘₐₓ = 8 µl, in particular vₘₐₓ = 5µl, in particular with vₘₐₓ = 3 µl. The liquid sample is directly filled into the wells.

It is in particular not necessary to coat a preferred aluminum sample holder because the adsorption of DNA on the aluminum surface is prevented by the presence of proteins, e.g. bovine serum albumin (BSA) in the sample volumes. Such proteins are standard in PCR reagents.

Advantageously, the wells are cylindrically shaped. In further embodiments, the wells can further have a hemispherical shape, a conical shape, or an elliptical cone shape. The shape of the wells refers to the shape that the wells form in the material.

In particular, at least a large part of the bottom area of the well is flattened, but it can further have a convex or concave area. The specific bottom area of the wells is preferably adapted to optimally reflect an optical signal.

The spacing between the wells of the array can be varied and is in particular a result of the density and size of the particular array. Anyway, the spacing between the individual wells should be large enough to prevent interferences between the individual sample volumes captured in the wells.

The advantage of a metallic sample holder lies further in its reflective properties that simplifies the detection of any light signal generated within the sample volume during the chemical or biochemical reaction.

Advantageously, the sample holder is made of aluminum, silver, gold, copper, or alloys thereof. In a further preferred embodiment, the sample holder might be made out of steel.

The material for the sample holder is advantageously chosen to have a high thermal conductivity for good thermal transport and/or high stiffness for a high stability of the shape of the sample holder.

An advantageous embodiment of the sample holder can further comprise a non-metallic covering sheet for covering the array of wells. The covering sheet in particular avoids evaporation of the sample volumes captured in the wells. The covering sheet might be fabricated out of any material that does not interfere with the sample volume. The sheet might have a hydrophobic character or comprise a hydrophobic layer. It might be deformable, e.g. like silicon rubber, or might be rigid, e.g. like polymeric glass. The sheet is preferably releasable from the sample holder.

In a further advantageous embodiment, the wells are covered by means of a hydrophobic medium for preventing evaporation of the sample volumes. Such medium might be an inert liquid that is non-miscible with water and does not react with the sample holder material or the sample volumes. An example for such a medium might be oil, in particular mineral oil or silicone oil.

A **second aspect** of the invention refers to a probe for performing quantitative polymerase chain detection reactions on the metallic sample holder. In particular, at least parts of the probe are coated with a fluorophore.

A **third aspect** of the invention refers to an apparatus, in particular for polymerase chain reaction detection, adapted for receiving the metallic sample holder according to the first aspect of the invention, comprising a thermal setting element thermally coupleable to the sample holder for controlling the temperature of the sample holder. In addition, the apparatus comprises a controller for controlling a thermal cycle of the thermal setting element. In addition the apparatus comprises an optical detector arranged in line of sight of the array of wells of the sample holder. The optical detector is configured to detect a plurality of optical signals from the sample volume of each well of the first sector of the array, and to detect a plurality of optical signals from the sample volume of each well of the second sector of the array of the metallic sample holder.

In an advantageous embodiment of the invention, the apparatus comprises the sample holder according to the first aspect of the invention.

Advantageously, the optical detector detects all signals from each well at the same time.

If the thermal setting element is coupled to the sample holder, it heats or cools down the sample holder. It can therefore be defined as a "heating and/or cooling element". The thermal setting element can work in the range of 10-120°C, preferably it works in the range of 40-100°C.

The thermal setting element can comprise a stage for mounting the sample holder. In particular such a stage could comprise an attachment element, e.g. a clip, to fix the sample holder to the stage, such that good thermal transport is achieved between the sample holder and the thermal setting element, respectively its stage.

Preferably, the thermal setting element is a Peltier thermoelectric device. Such a Peltier device can be constructed of pellets of n-type and p-type semiconductor material that are alternately placed in parallel to each other and are electrically connected in series. Examples of semiconductor materials that can be utilized to form the pellets in a Peltier device, include but are not limited to, bismuth telluride, lead telluride, bismuth selenium and silicon germanium. However, it should be appreciated that the pellets can be formed from any semiconductor material as long as the resulting Peltier device exhibits thermoelectric heating and cooling properties when a current is run through the Peltier device. In various embodiments, the interconnections between the pellets can be made with copper which can be bonded to a substrate. Examples of substrate materials that can be used include but are not limited to copper, aluminum, aluminum nitride, beryllium oxide, polyimide or aluminum oxide. In various embodiments the substrate material can include aluminum oxide also known as alumina. It should be understood, however, that the substrate can include any material that exhibits thermally conductive properties.

In a further advantageous embodiment, the controller of the apparatus is configured to control the optical detector for recording a plurality of optical signals from a plurality of sample volumes of a plurality of wells for each thermal cycle of the thermal setting element.

In a further advantageous embodiment of the apparatus, the optical detector is configured to assign each optical signal to the corresponding sample volume, and in particular to the corresponding first or second sector.

Advantageously, the apparatus recognizes the first and the second sector of the array. By allocating the respective optical signal to the first and/or second sector of the array, the apparatus can detect, whether a respective first and/or second nucleic acid is present in the respective well of the array.

In particular, the sample holder is thermally very well coupled to the thermal setting element, such that the thermal conductivity between the holder and the element is very high. To achieve the high thermal conductivity between the two, the sample holder, when mounted to the apparatus, is pressed against the thermal setting element, in particular by means of a clamp mechanism.

For a further advantageous embodiment of the apparatus, the thermal interface conductance between the thermal setting element and the sample holder is at least 1000 W/(m²K), in particular at least 4000 W/(m²K), in particular at least 8000 W/(m²K).

A preferred embodiment of the apparatus comprises a body. The body of the apparatus is in particular defined as being a shell or a housing that surrounds all the components of the apparatus. The body can therefore be a metallic shell or a metallic scaffold that holds the components together.

A further advantageous embodiment of the apparatus comprises at least one temperature sensor for sensing the temperature of the sample holder, in particular, wherein the temperature sensor is connected to the controller and the thermal setting element for providing a feedback loop for controlling the temperature of the sample holder,

In an further advantageous embodiment of the invention, the controller is configured to steer the thermal setting element to heat the sample holder) with a net effective heating ramp equal or higher than 5.0°C/s, in particular equal or higher than 8.0°C/s, in particular equal or higher than 10.0°C/s, and/or wherein the controller is configured to steer the thermal setting element to cool down the sample holder with a net effective cooling ramp equal or lower than -5.0°C/s, in particular equal or lower than -8.0°C/s, in particular equal or lower than - 10.0°C/s.

In a further advantageous embodiment, the apparatus comprises a body, in particular a metallic body, wherein the metallic sample holder is thermally coupleable to the body.

In a further advantageous embodiment of the invention, the apparatus consists of at least 80 wt% metal, in particular of at least 90 wt% metal, wherein in particular the metal is aluminum.

In a further advantageous embodiment of the invention, the apparatus is portable, in particular being less than 3 kg of weight.

A third aspect of the invention refers to a use of the sample holder according to the first aspect for the polymerase chain reaction detection of sample volumes, in particular by means of the apparatus according to the second aspect of the invention.

A **fourth aspect** of the invention refer to a method for detection of a first or a second nucleic acid from a sample and/or for distinction of the first nucleic acid from the second nucleic acid of the sample, by means of the metallic sample holder according the first aspect, comprising the steps of
- application of the nucleic acids isolated from the sample onto the first sector and the second sector of the array of the sample holder and thereby dissolving the freeze-dried nucleic acid amplification test kit,
- running a temperature program with the apparatus according to claims 15 to 24,
- detection of the presence or absence of the first and/or second nucleic acid.

A **fifth aspect** of the invention refers to a use of the apparatus, wherein the apparatus comprises a camera for monitoring the method steps. A feedback loop informs an operator of the apparatus about whether the method steps have been conducted properly. E.g. if the user forgets to prepare the sample holder properly, the camera might detect such failure and warn the user. In a further embodiment, the camera might read a unique identification symbol on the sample holder, e.g. a QR-code or serial number, for identification of the sample holder at the same time as the camera monitors the processing of the sample holder.

In a further advantageous embodiment of the invention, the apparatus is adapted to support a user by means of a controller that monitors the method steps performed by the user and that sends signals and results generated by the apparatus to the user.

Other advantageous embodiments are listed in the dependent claims as well as in the description below.

### Brief Description of the Drawings

The invention will be better understood and objects other than those set forth above will become apparent from the following detailed description thereof. Such description makes reference to the annexed drawings, wherein:
Fig. 1 shows a schematic of a metallic sample holder according to an embodiment of the invention,
Fig. 2a shows an enlarged section of the sample holder,
Fig. 2b shows a schematic of the metallic sample holder with the first and second sector according to an embodiment of the invention;
Fig. 3 shows a schematic of a cross section of an apparatus according to an embodiment of the invention,
Fig. 4 shows a schematic perspective view of an apparatus according to an embodiment of the invention,
Fig. 5 shows a schematic view of a cross section of an apparatus according to an embodiment of the invention.

### Modes for Carrying Out the Invention

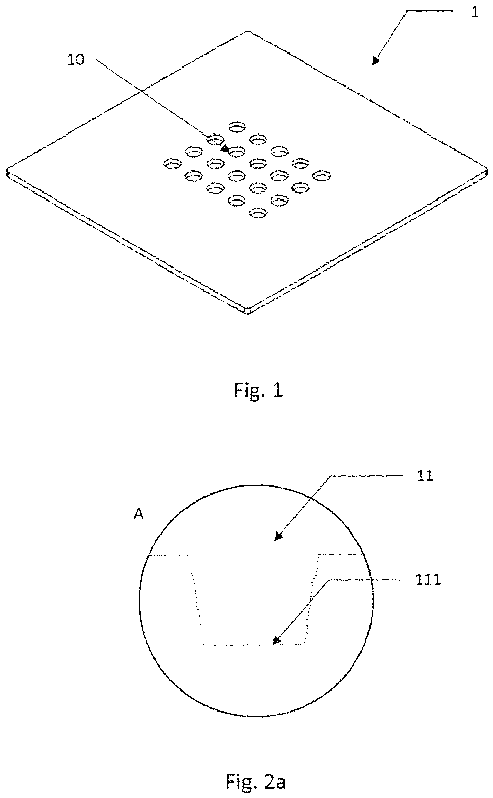

Fig. 1 shows a schematic view of a preferred embodiment of the metallic sample holder 1 according to the invention. The sample holder 1 comprises an array of wells 10. The array 10 can cover selected areas of the sample holder 1 as shown in the figure or can extend over the full area of the sample holder 1. In particular, the wells 11 of the array 10 can be arranged in a specific pattern. In the embodiment shown in the figure, the wells 11 are arranged evenly spaced in a regular rectangular pattern.

A preferred embodiment of the invention comprises an array 10 with at least 2-3 wells. The array of wells 10 displayed in the figure showing only few wells 11 is a symbolic illustration of the preferred array 10.

The metallic sample holder 1 is preferably made of aluminum.

A preferred size of the sample may be 4cm times 4cm. The small size and the correlating small mass of the sample holder allows fast heating and cooling of the holder and therefore faster thermal cycling. In particular, the arrangement of the array 10 on the sample holder 1 leaves space at the edge of the sample holder 1 to handle the holder 1 by means of hands or tweezers without interfering with the array of wells 10. Preferably, no wells 11 are arranged within a distance of 0.5 cm from the edge of the sample holder 1.

As shown in the enlarged section in Fig. 2a, the wells 11 themselves are essentially of cylindrical shape. The essentially cylindrical shape can also tend to be slightly conical, for fabrication reasons, as shown in the figure.

In particular, the wells 11 have an at least partially flat bottom area 111. The essentially flat bottom area 111 serves for reflecting an optical signal. In particular because the sample holder 1 is made of metal, the reflected optical signal is very strong and can be detected by an optical detector 4.

The sample volumes are directly filled into the wells. No coating of the metallic sample holder 1 is required.

In particular, the well 11 has a flat bottom surface 111 that promotes reflection of the optical signal within the well 11.

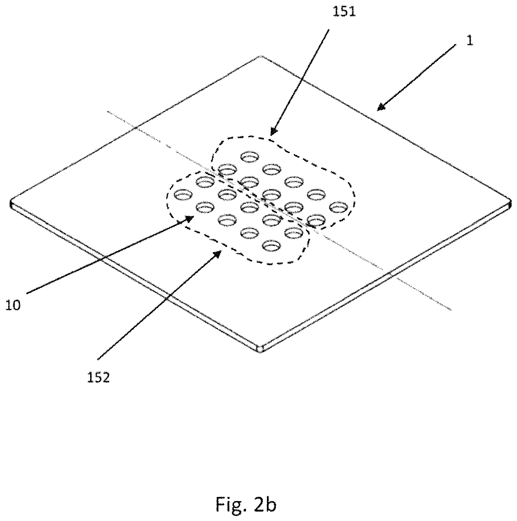

Fig. 2b schematically shows the separation of the array into a first sector 151 and a second sector 152, wherein the first sector 151 of the array comprises wells that are loaded with a first freeze-dried nucleic acid amplification test kit. Wherein the second sector 152 of the array comprises wells that are loaded with a second freeze-dried nucleic acid amplification test kit.

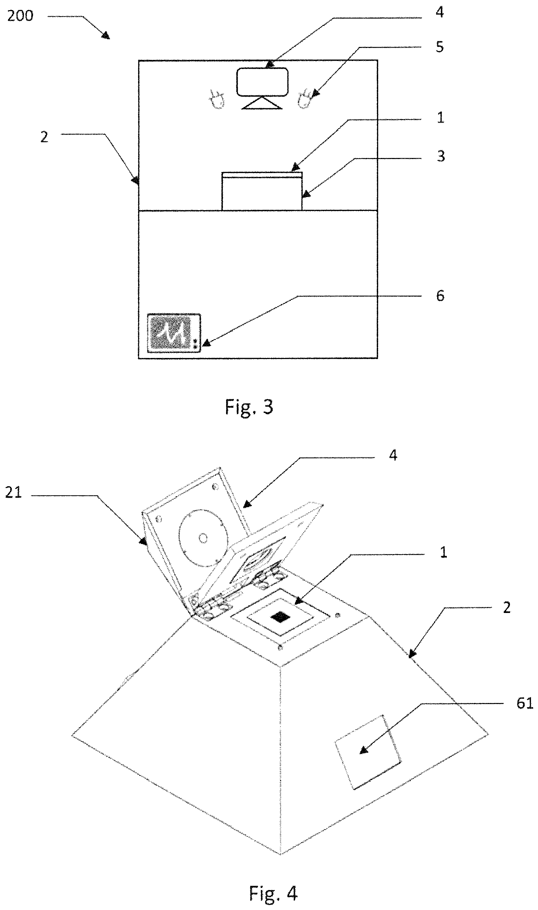

Fig. 3 shows a cross-section of a preferred embodiment of the apparatus 200. The apparatus 200 comprises a body 2. In the preferred embodiment shown in the figure, all components of the apparatus 200 are arranged within this body 2.

The apparatus 200 is adapted for receiving the metallic sample holder 1. In the figure, the apparatus 200 is illustrated comprising the sample holder 1. The apparatus 200 comprises a thermal setting element 3 that is thermally coupled to the sample holder 1 for controlling the sample holder 1 temperature. In the figure, the sample holder 1 is arranged on the thermal setting element 3.

In addition, the apparatus 200 further comprises a controller 6 for controlling the thermal cycle of the thermal setting element 3. The controller 6 might be arranged in a base body of the apparatus 200 as shown in Fig. 3. The controller 6 might comprise a screen 61 for displaying the cycling data. In particular, the controller 6 sends signals to the thermal setting element 3 for steering the heating or cooling of the thermal setting element 3. Since the thermal setting element 3 is thermally coupled to the sample holder 1, the sample holder 1 reaches the same temperature as the thermal setting element 3.

Therefore, the temperature of the sample holder 1 can be controlled by the controller 3. In addition, the temperature cycles of the sample holder 1 can also be controlled by the controller 3.

For example, three consecutive heating and cooling cycles are performed. Initially, the sample holder 1 is heated for 9 seconds, then it is kept at a temperature above 80°C for 14 seconds and then it is cooled for 6.5 seconds. Preferably, the system is switched off for 10 seconds after every heating and cooling cycle.

The apparatus comprises further an optical detector 4 arranged in a way that the array of wells 10 of the sample holder 1, if there is any sample holder 1 mounted to the apparatus 200, would be in line of sight of the optical detector 4. In line of sight of the optical detector 4 refers to the arrangement of the optical detector 4 in an optical line with the array of wells 10, as shown in Fig. 3, where the optical detector 4 is arranged in an upper part of the body 2 of the apparatus 200. The thermal setting element 3 that preferably comprises a stage for the sample holder 1 is arranged in a centre part of the body 2 of the apparatus 200.

The distance between the level of the sample holder 1 that is mounted to the stage of the thermal setting element 3 and the level of the optical detector 4 is about 4.5 cm.

Not visible on the figures, a lens could be arranged between the sample holder 1 and the optical detector 4.

The optical detector 4 is configured to detect at least one optical signal from one sample volume of one well 11 of the sample holder 1.

The optical signal that can be detected by the optical detector 4 is preferably an optical signal that is generated by the liquid sample volume if the sample volume is excited by light that is reflected on the surface area, in particular on the flat bottom area 111, of one of the wells 11.

Preferably, the optical detector 4 receives an optical signal from each sample volume of the wells 11 of the array 10 simultaneously.

The optical detector 4 comprises preferably a CCD or CMOS sensor as an optical sensor element. In particular, the optical detector 4 can comprise a lens for focussing the optical signal to the optical sensor element.

The apparatus 200 can comprise a light source 5 for exciting the sample volume. The light source 5 might be an individual lamp. Preferably, the light source 5 is designed as a set of lamps arranged close to and/or around the optical detector 4. The light source 5 might be one or more LEDs or a mercury lamp.

Fig. 4 shows a perspective view of an embodiment of an apparatus 200 according to the invention. The sample holder 1 is mounted to the apparatus 200.

Preferably, the sample holder 1 is mounted to the thermal setting element 3 or in particular to a stage of the thermal setting element 3 of the apparatus 200. In a further embodiment, the sample holder 1 could also be mounted to a stage of the apparatus 200, wherein the thermal setting element 3 is in thermal connection to the sample holder 1 for heating or cooling the sample holder 1 and the sample volumes respectively.

The body 2 serves as a housing and also as a scaffold for all the components of the apparatus 200.

The body 2 has preferably a pyramidal outer shape as shown in the figure. This shape of the body 2 is advantageous, since it has a bigger surface for a given machine volume and therefore its functionality as a heat sink is very efficient.

In particular the sample holder 1, if mounted to the apparatus 200 as shown in this figure, is thermally coupled to the body 2, such that the body 2 might serve as a thermal heat sink for enabling fast change of temperature of the sample holder 2.

The optical detector 4 and any optional lenses are arranged within a peak section 21 of the preferably pyramidal body 2. In addition, also the light source 5 can be arranged within this peak 21.

To mount the sample holder 2 to the apparatus 200, the peak section 21 can be lifted as shown in the figure and the sample holder 1 can be arranged onto the thermal setting element 3 or a stage of the thermal setting element 3.

The body 2 of the apparatus 200 can further comprise a screen 61, e.g. to display temperature data of the thermal setting element 3 or of a temperature sensor.

Preferably, the apparatus 200 has the dimensions of 8cm X 8cm X 17cm and is very lightweight. Preferably, the apparatus 200 weights less than 5kg, in particular less than 3kg.

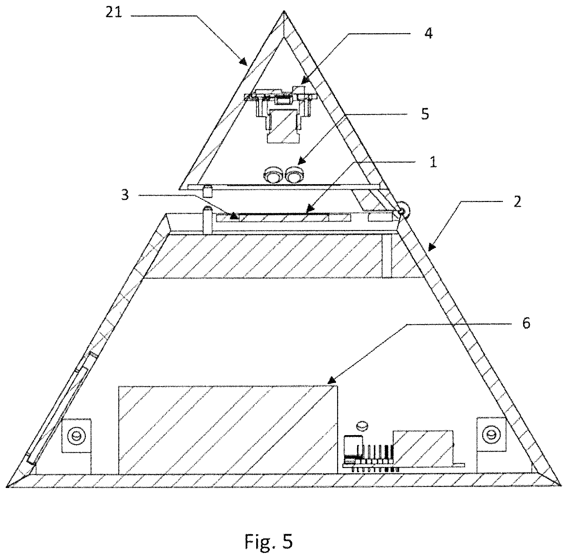

Fig. 5 shows a cross section of a preferred embodiment of the apparatus 200. The sample holder 1 is mounted to the apparatus 200.

The cross-sectional view reveals the interior of the apparatus 200. In particular, the optical detector 4 and the light source 5 are arranged in the peak section 21 of the apparatus body 2.

The sample holder 1 is mounted to the thermal setting element 3.

The light source 5 in this embodiment is arranged close by the location, in particular a stage, where the sample holder 1 is mounted.

The peak section 21 can be lifted for mounting the sample holder 1 onto the stage. In particular, the peak section 21 might be connected to the remaining body 2 by means of a hinge.

A controller 6 is arranged within the lower part, respectively the remaining part without the peak section 21, of the apparatus body 2.

### Examples

### Example 1:

The first example was performed with a sample holder with dimensions of 40x40x8 mm. The wells had a diameter of 2mm, a depth of 0.6mm and the sample holder is made of aluminum.

The detection capabilities of the device have been investigated by analyzing a sample volume comprising:
- Mastermix: gGeneon One.Direct.Step RT-qPCR Kit for Probes (2X), which contains Hot Start Taq DNA Polymerase, reverse transcriptase, reaction buffer, dNTPs.
- A solution of bovine serum albumin and MgSO4
- Deionized water

The first section testing for a coronavirus had the following primers and probes:
- A forward primer: GAC CC C A AA ATC AG C G AA AT
- A reverse primer: TCT GG T T AC TGC CA G T TG AAT CT G
- A fluorecent probe : [FAM] ACC CC G C AT TAC GT T T GG TGG AC C[BHQ-1]

A positive control single strand RNA template: EURM-019 Sequence (5'-3'):

GGGAGACGAAUUGGGCCCUCUAGAUGCAUGCUCGAGCGGCCGCCAGUGUGAUGGAUAUC
UGCAGAAUUCGCCCUUAUUCAAGUAUUGAGUGAAAUGGUCAUGUGGCGGUUCACUAU
AUGUUAAACCAGGUGGAACCUCAUCAGGAGAUGCCACAACUGCUUAUGCUAAUAGUGUU
UUUAACAUUUGUCAAGCUGUCCGGAAGAGACAGGUACGUUAAUAGUUAAUAGCGUACUU
CUUUUUCUUGCUUUCGUGGUAUUCUUGCUAGUUACACUAGCCAUCCUUACUGCGCUUCG
AUUGUGUGCGUACUGCUGCAAUAUUGUUAACGUAUAAUGGACCCCAAAAUCAGCGAAAU
GCACCCCGCAUUACGUUUGGUGGACCCUCAGAUUCAACUGGCAGUAACCAGAAUGGAGA
ACGCAUUGCAACUGAGGGAGCCUUGAAUACACCAAAAGAUCACAUUGGCACCCGCAAUC
CUGCUAACAAUGCUGCAAUCGUGCUACAACUUCCUCAAGGAAUUUUGGGACCAGGAA
CUAAUCAGACAAGGAACUGAUUACAAACAUUGGCCGCAAAUUGCACAAUUUGCCCCCAG
CGCUUCAGCGUUCUUCGGAAUGUCGCGCAUUGGCAUGGAAGUCACACCUUCGGGAACGU
GGUUGACCUACACAGGUGCCAUCAAAUUGGAGUGUGACAUACCCAUUGGUGCAGGUAUA
UGCGCUAGUUAUCAGACUCAGACUAAUUCUCCUCGGCGGGCACGUAGUGUAGCUAGUCA
ACCUGCUUUUGCUCGCUUGGAUCCGAAUUCAAAGGUGAAAUUGUUAUCCGCUCACAAUU
CCACACAACAUACGAGCCGGAAGCAUAAAGUGUAAAGCCUGGGGUGCCUAAUGA.

The second section for an influenzavirus had the following primers and probes:
- A forward primer: CAA GA C C AA TCY TG T C AC CTC TG A C
- A reverse primer: GCA TT Y T GG ACA AA V C GT CTA CG
- fluorecent probe : [FAM] TGC AGT CCT CGC TCA CTG GGC ACG [BHQ-1]

A positive control single strand RNA template: CAA GAC CAA UCC UGU CAC CUC UGA CUA AGG GGA UUU UAG GGU UUG UGU UCA CGC UCA CCG UGC CCA GUG AGC GAG GAC UGC AGC GUA GAC GGU UUG UCC AAA ACG C.
A sample volume smaller than 5 µL has been applied onto each well. The sample holder has been placed under the optical detector and the excitation light source of the device. The excitation light source is composed of 8 commercial LEDs with a power rating of 3W and a central wavelength of 460nm. The light sources were directed to the sample holder for exciting the sample volumes captured in the wells of the sample holder array.

Detection was performed with a single-board computer (Raspberry Pi model 3B) equipped with a CCD camera (Raspberry Pi Camera Module v2) that was placed vertically above the center of the sample holder. A 12mm lens was mounted onto the CCD camera and a long pass filter was placed in front of the lens (cut on frequency approximately at 580 nm). The signal was detected by recording the image produced by the CCD camera and saving it to an image file. Portion of the signal detected by the optical sensor is shown in Fig. 6b.
While there are shown and described presently preferred embodiments and examples of the invention, it is to be distinctly understood that the invention is not limited thereto but may be otherwise variously embodied and practised within the scope of the following claims.

### Example 2:

In a further example, the NAAT reaction system may include: a nucleic acid amplification enzyme such as a DNA polymerase or a RNA polymerase, a reverse transcriptase enzyme, trehalose, bovine serum albumin, Tris-Cl, dNTPs, MgSO₄.

Advantageously, the nucleic acid detection probes are functionalized with a fluorophore, for example FAM or ROX at the 5' end and a fluorescence quencher, for example BHQ-1 at the 3' end. The NAAT reaction system includes: forward primer 0.335µM, reverse primer 0.335µM, TaqMan probe 0.085µM, DNA polymerase 0.5U/uL, reverse transcriptase 0.5U / µL; dNTPs 0.3mM; Mg²⁺ 3mM; seaweed Sugar 5µM; Tris-Cl 5mM. The total volume is 40µL and the volume for each well is 2µL. The reaction mix is fixed in individual wells through a freeze drying process. Not all wells are filled with the same reaction mix. There are at least 4 wells with primer/probe combinations for N1, at least 4 wells with primer/probe combinations for N2, at least 4 wells with primer/probe combinations for a control such as RnaseP, at least 4 wells with primer/probe combinations for the first virus (e.g. an first influenza virus) and at least 4 wells with primer/probe combinations for the second virus (e.g. a second influenza virus). At least 1 well of each primer/probe combination contains a positive control which may be a plasmid construct with known concentration.

## Claims

1. A metallic sample holder (1), comprising
- an array of wells (10),
- wherein each well of the array is adapted to capture a sample volume,
- wherein a first sector (151) of the array comprises wells that are loaded with a first freeze-dried nucleic acid amplification test kit.
- wherein a second sector (152) of the array comprises wells that are loaded with a second freeze-dried nucleic acid amplification test kit.

2. The metallic sample holder (1) according to claim 1 is adapted for capturing sample volumes for nucleic acid amplification test reactions.

3. The metallic sample holder (1) according to one of the preceding claims, wherein the first test kit comprises a first set of primers specific for a first test for identifying and/or quantifying a first nucleic acid and wherein the second test kit comprises a second set of primers specific for a second test for identifying and/or quantifying a second nucleic acid.

4. The metallic sample holder (1) according to claim 3,
wherein the first test kit comprises a nucleic acid amplification enzyme, dNTPs and a buffer; and/or
wherein the second test kit comprises a nucleic acid amplification enzyme, dNTPs and a buffer.

5. The metallic sample holder (1) according to one of the preceding claims,
wherein the first test kit comprises a nucleic acid detection probe for a first test for identifying and/or quantifying a first nucleic acid and/or a fluorescent dye; and/or
wherein the second test kit comprises a nucleic acid detection probe for a second test for identifying and/or quantifying a second nucleic acid and/or a fluorescent dye.

6. The metallic sample holder (1) according to one of the preceding claims,
wherein the first test kit comprises a reverse transcriptase enzyme; and/or
wherein the second test kit comprises a reverse transcriptase enzyme.

7. The metallic sample holder (1) according to claims 1-5 and/or claim 6,
wherein the first nucleic acid is a first RNA or DNA isolated from a first virus, in particular from a corona virus; and/or
wherein the second nucleic acid is a second RNA or DNA isolated from a second virus, in particular an influenza virus.

8. The metallic sample holder (1) according to claims 1-5 and/or claim 6,
wherein the first nucleid acid is a first RNA or DNA isolated from a first virus, in particular from the SARS-Co-2 virus; and/or
wherein the second nucleic acid is a second RNA or DNA isolated from a second virus, in particular an influenza virus.

9. The metallic sample holder (1) according to one of the preceding claims, wherein each well (11) is adapted to capture a maximal sample volume vₘₐₓ, with vₘₐₓ = 15 µl, in particular with vₘₐₓ = 8 µl, in particular with vₘₐₓ = 5 µl, in particular with vₘₐₓ = 3 µl, and/or
wherein the metallic sample holder (1) consists of aluminum, silver, gold, copper, or alloys thereof.

10. The metallic sample holder (1) according to one of the preceding claims, wherein each well (11) is of cylindrical shape, of conical shape, or of elliptical shape and/or has at least partially a flat bottom area, and/or
wherein the bottom area is configured to reflect an optical signal by means of a flat metallic layer, and/or
wherein the surface of the wells is free of any coating except nucleic acid amplification test kits.

11. An apparatus (200), in particular for polymerase chain reaction detection, adapted for receiving the metallic sample holder (1) according to any one of claims 1 to 10, comprising
- a thermal setting element (3) thermally coupleable to the sample holder (1) for controlling the temperature of the sample holder (1),
- a controller (6) for controlling a thermal cycle of the thermal setting element (3)
- an optical detector (4) arranged in line of sight of the array of wells (10) of the sample holder (1), wherein the optical detector (4) is configured to detect a plurality of optical signals from the sample volume of each well of the first sector (151) of the array, and to detect a plurality of optical signals from the sample volume of each well of the second sector (152) of the array of the metallic sample holder.

12. The apparatus (200) according to claim 11, wherein the controller (6) is configured to control the optical detector (4) for recording a plurality of optical signals from a plurality of sample volumes of a plurality of wells (11) for each thermal cycle of the thermal setting element (3), and/or
wherein the optical detector (4) is configured to assign each optical signal to the corresponding sample volume, and in particular to the corresponding first or second sector.

13. The apparatus (200) according to any one of the preceding claims 11 to 12,
wherein the thermal interface conductance between the thermal setting element (3) and the sample holder (1) is at least 1000 W/(m²K), in particular at least 4000 W/(m²K), in particular at least 8000 W/(m²K), and/or
wherein the controller is configured to steer the thermal setting element (3) to heat the sample holder (1) with a net effective heating ramp equal or higher than 5.0°C/s, in particular equal or higher than 8.0°C/s, in particular equal or higher than 10.0°C/s, and/or
wherein the controller is configured to steer the thermal setting element (3) to cool down the sample holder with a net effective cooling ramp equal or lower than -5.0°C/s, in particular equal or lower than -8.0°C/s, in particular equal or lower than -10.0°C/s.

14. A method for detection of a first or a second nucleic acid from a sample and/or for distinction of the first nucleic acid from the second nucleic acid of the sample, by means of the metallic sample holder according to claims 1 to 10, comprising the steps of
- application of the nucleic acids isolated from the sample onto the first sector and the second sector of the array of the sample holder and thereby dissolving the freeze-dried nucleic acid amplification test kit running a temperature program with the apparatus according to claims 14 to 13,
- detection of the presence or absence of the first and/or second nucleic acid.

15. Use of the apparatus according to any one of claims 11 to 13 for the method according to claim 14, wherein the apparatus comprises a camera for monitoring the method steps, wherein a feedback loop informs an operator of the apparatus about whether the method steps have been conducted.


SEQUENCE LISTING

<110> ETH Transfer

<120> A Metallic Sample Holder, a Probe for Performing Detection Reactions,
and Apparatus for receiving the Sample Holder

<130> P183731EP00

<160> 8

<170> BiSSAP 1.3.6

<210> 1
<211> 20
<212> DNA
<213> Artificial Sequence

<220>
<223> forward primer coronavirus

<400> 1
gaccccaaaa tcagcgaaat                                                    20

<210> 2
<211> 24
<212> DNA
<213> Artificial Sequence

<220>
<223> reverse primer coronavirus

<400> 2
tctggttact gccagttgaa tctg                                               24

<210> 3
<211> 24
<212> DNA
<213> Artificial Sequence

<220>
<223> 5′-FAM, 3′-BHQ-1; probe coronavirus

<220>
<221> modified_base
<222> 1
<223> /mod_base="OTHER"
      /note="Carboxyfluorescein [FAM] - Adenosine "

<220>
<221> modified_base
<222> 24
<223> /mod_base="OTHER"
      /note="Black Hole Quencher-1 [BHQ-1] - cytidine"

<400> 3
accccgcatt acgtttggtg gacc                                               24

<210> 4
<211> 880
<212> RNA
<213> SARS coronavirus


<220>
<223> positive control single strand RNA Coronavirus - EURM-019

<400> 4
gggagacgaa uugggcccuc uagaugcaug cucgagcggc cgccagugug auggauaucu      60

gcagaauucg cccuuauuca aguauugagu gaaaugguca uguguggcgg uucacuauau      120

guuaaaccag guggaaccuc aucaggagau gccacaacug cuuaugcuaa uaguguuuuu      180

aacauuuguc aagcuguccg gaagagacag guacguuaau aguuaauagc guacuucuuu      240

uucuugcuuu cgugguauuc uugcuaguua cacuagccau ccuuacugcg cuucgauugu      300

gugcguacug cugcaauauu guuaacguau aauggacccc aaaaucagcg aaaugcaccc      360

cgcauuacgu uugguggacc cucagauuca acuggcagua accagaaugg agaacgcauu      420

gcaacugagg gagccuugaa uacaccaaaa gaucacauug gcacccgcaa uccugcuaac      480

aaugcugcaa ucgugcuaca acuuccucaa ggaaauuuug gggaccagga acuaaucaga      540

caaggaacug auuacaaaca uuggccgcaa auugcacaau uugcccccag cgcuucagcg      600

uucuucggaa ugucgcgcau uggcauggaa gucacaccuu cgggaacgug guugaccuac      660

acaggugcca ucaaauugga gugugacaua cccauuggug cagguauaug cgcuaguuau      720

cagacucaga cuaauucucc ucggcgggca cguaguguag cuagucaacc ugcuuuugcu      780

cgcuuggauc cgaauucaaa ggugaaauug uuauccgcuc acaauuccac acaacauacg      840

agccggaagc auaaagugua aagccugggg ugccuaauga                            880


<210> 5
<211> 25
<212> DNA
<213> Artificial Sequence


<220>
<223> forward primer influenzavirus

<400> 5
caagaccaat cytgtcacct ctgac                                            25


<210> 6
<211> 23
<212> DNA
<213> Artificial Sequence


<220>
<223> reverse primer influenzavirus

<400> 6
gcattytgga caaavcgtct acg                                              23

<210> 7
<211> 24
<212> DNA
<213> Artificial Sequence

<220>
<223> 5′-FAM, 3′-BHQ-1; probe influenzavirus

<220>
<221> modified_base
<222> 1
<223> /mod_base="OTHER"
      /note="Carboxyfluorescein [FAM] - deoxythymidine"

<220>
<221> modified_base
<222> 24
<223> /mod_base="OTHER"
      /note="Black Hole Quencher-1 [BHQ-1] - guanosine"

<400> 7
tgcagtcctc gctcactggg cacg                                             24

<210> 8
<211> 106
<212> RNA
<213> Artificial Sequence

<220>
<223> positive control single strand RNA influenzavirus

<400> 8
caagaccaau ccugucaccu cugacuaagg ggauuuuagg guuuguguuc acgcucaccg     60

ugcccaguga gcgaggacug cagcguagac gguuugucca aaacgc                    106